# EUROPÄISCHE PATENTSCHRIFT

(11) **EP 2 543 406 B1**
(45) Veröffentlichungstag und Bekanntmachung des Hinweises auf die Patenterteilung: **11.05.2016**
(21) Anmeldenummer: 12173778.7
(22) Anmeldetag: 27.06.2012
(51) Int. Cl.: A61B 5/09, G01F 1/06, A61M 16/00, A61M 16/01, A61M 16/10, A61M 16/22, A61B 5/091

(54) **Beatmungssystem**
Ventilation system
Système de respiration

(30) Priorität: 02.07.2011 DE 102011106413; 05.11.2011 DE 102011117786
(43) Veröffentlichungstag der Anmeldung: 09.01.2013
(73) Patentinhaber: Drägerwerk AG & Co. KGaA, 23558 Lübeck (DE)
(72) Erfinder: Heesch, Ralf, 23568 Lübeck (DE); Rahlf, Till, 23617 Stockelsdorf (DE)

(56) Entgegenhaltungen:
- EP-A1- 0 621 049
- US-A- 5 810 002
- US-A- 6 095 137
- US-A1- 2005 133 033
- US-A1- 2011 061 650

## Beschreibung

Die vorliegende Erfindung betrifft ein Beatmungssystem mit einer Rückatemleitung und einem Reservoir.

Ein derartiges Beatmungssystem ist durch die Gebrauchsanweisung "Zeus Infinity Empowered" der Dräger Medical GmbH bekannt.
Dabei sind solche Beatmungssysteme mit einer Rückatemleitung in der Regel so aufgebaut, dass ausgehend von einem Patientenanschluss oder Y-Stück, über den/das ein Patient mit dem Beatmungssystem verbunden wird, ein inspiratorischer Zweig und ein exspiratorischer Zweig vorgesehen sind, deren von dem Patientenanschluss entfernte Enden über die Rückatemleitung, die in dem Gehäuse des Beatmungssystems verläuft, miteinander verbunden sind.
Dabei sind in der Rückatemleitung in der Regel ein Reservoir wie ein Handbeatmungsbeutel zur Aufnahme von Atemgas, ein CO₂-Absorber und ein Beatmungsantrieb, vorzugsweise in Form eines Radialverdichters, vorgesehen, mit dem Atemgas in den inspiratorischen Zweig gefördert werden kann.

Außerdem sind in dem inspiratorischen und dem exspiratorischen Zweig jeweils Rückschlagventile angeordnet, die verhindern, dass Atemgas, das von dem Patienten ausgeatmet wird, den inspiratorischen Zweig verlässt bzw. dass Atemgas von der Rückatemleitung aus direkt in den exspiratorischen Zweig gedrückt werden kann. Schließlich ist nahe der Verbindung zwischen dem exspiratorischen Zweig und der Rückatemleitung ein Steuerventil oder PEEP-Ventil angeordnet, das von einer Steuerung geöffnet oder geschlossen werden kann.

Während der Inspirationsphase ist das Steuerventil geschlossen, um zu vermeiden, dass Atemgas durch den Beatmungsantrieb direkt wieder in die Rückatemleitung gedrückt wird, anstatt in den Patientenanschluss gedrückt zu werden. Während der Exspirationsphase ist das Steuerventil jedoch geöffnet, so dass das aus dem Patientenanschluss ausgestoßene Atemgas aus dem Exspirationszweig zurück in die Rückatemleitung gelangen kann, wobei dieses Atemgas zunächst in das Reservoir, also in der Regel den Handbeatmungsbeutel, gelangt.

Im normalen Beatmungsbetrieb wird Atemgas damit zunächst während der Inspirationsphase mit Hilfe des Beatmungsantriebs aus dem Reservoir gezogen und über den inspiratorischen Zweig dem Patientenanschluss zugeführt, wobei während dieser Phase das Steuerventil geschlossen ist. Während der Exspirationsphase wird das Steuerventil geöffnet, und das ausgeatmete Gas gelangt wieder zurück in das Reservoir.

Hierbei kann sich allerdings das Problem ergeben, dass das Reservoir bzw. der Handbeatmungsbeutel ein zu kleines Volumen aufweist. Dann wird ein Teil des von dem Patientenanschluss kommenden Gases über ein in der Rückatemleitung vorgesehenes Narkosegasfortleitungsventil (im Folgenden "NGF-Ventil") ausgestoßen. Das hat dann allerdings zur Folge, dass für die nächste Inspirationsphase nicht genügend Gas im System vorhanden ist und dies durch eine Zuführung von frischen Gasen kompensiert werden muss. Damit kann das Beatmungssystem dann nicht in der eigentlich vorgesehenen Weise betrieben werden, bei der nur sehr wenig kostspielige Gase neu zugeführt werden müssen.

Ferner ist aus dem Stand der Technik, wie beispielsweise der US 5,810,002, ein Beatmungssystem bekannt mit einem Patientenanschluss, mit einem inspiratorischen Zweig und einem exspiratorischen Zweig, die beide mit dem Patientenanschluss verbunden sind, mit einer Rückatemleitung, die den inspiratorischen Zweig mit dem exspiratorischen Zweig verbindet, mit einem Reservoir, das mit der Rückatemleitung verbunden ist, mit einem Steuerventil, das in der Rückatemleitung vorgesehen ist, und mit einer Steuereinheit, die mit dem Steuerventil verbunden ist.

Aus der US 2005/0133033 A1 ist ein Beatmungssystem bekannt mit einem Patientenanschluss, einem inspiratorischen und einem exspiratorischen Zweig und mit einer Rückatemleitung, die mit einem Reservoir verbunden ist, wobei zwischen Reservoir und Rückatemleitung eine Atemzugvolumen-Messvorrichtung, ein Steuerventil, zwei Drucksensoren und eine Steuereinheit, die mit der Atemzugvolumen-Messvorrichtung, dem Steuerventil und den Drucksensoren verbunden ist, vorgesehen sind.

Die US 2011/0061650 A1 und die US 6,095,137 offenbaren Anästhesievorrichtungen mit einem Patientenanschluss, einem inspiratorischen und einem exspiratorischen Zweig, einer Rückatemleitung, einem Reservoir, einem Steuerventil und einer Steuereinheit. Die US 6,095,137 offenbart zusätzlich noch einen Drucksensor, der neben dem Reservoiranschluss mit der Rückatemleitung verbunden ist.

Die EP 0 621 049 A1 beschreibt ein Beatmungssystem mit einem Patientenanschluss, einem inspiratorischen und einem exspiratorischen Zweig, mit einer Rückführung, die mit einer Steuereinheit verbunden ist, und mit einem Reservoir.

Daher ist es ausgehend vom Stand der Technik die Aufgabe der vorliegenden Erfindung, ein eingangs beschriebenes Beatmungssystem bereitzustellen, mit dem ein zu kleines Volumen des Reservoirs in der Rückatemleitung zuverlässig erkannt wird.

Erfindungsgemäß wird diese Aufgabe durch ein Beatmungssystem gelöst mit einem Patientenanschluss, einem inspiratorischen Zweig, der ein erstes und ein zweites Ende, sowie ein Rückschlagventil aufweist, das dann öffnet, wenn der Druck auf der zum zweiten Ende weisenden Seite des Rückschlagventils über dem auf der zum ersten Ende weisenden Seite des Rückschlagventils liegt, wobei das erste Ende mit dem Patientenanschluss verbunden ist, einem exspiratorischen Zweig, der ein erstes und ein zweites Ende, sowie ein Rückschlagventil aufweist, das dann öffnet, wenn der Druck auf der zum ersten Ende weisenden Seite des Rückschlagventils über dem auf der zum zweiten Ende weisenden Seite des Rückschlagventils liegt, wobei das erste Ende mit dem Patientenanschluss verbunden ist, einer Rückatemleitung, die das zweite Ende des inspiratorischen Zweigs mit dem zweiten Ende des exspiratorischen Zweigs verbindet, einem Reservoir,
das ein variables Volumen mit einem Maximalvolumen hat und das mit einem Reservoiranschluss in der Rückatemleitung verbunden ist, einem betätigbaren Steuerventil, das in der Rückatemleitung zwischen dem zweiten Ende des exspiratorischen Zweigs und dem Reservoiranschluss vorgesehen ist,
einem Drucksensor, der benachbart zu dem Reservoiranschluss mit der Rückatemleitung verbunden ist, und einer Steuereinheit, die mit dem betätigbaren Steuerventil und mit dem Drucksensor verbunden ist, wobei
das Beatmungssystem angepasst ist, um die folgenden Schritte auszuführen:
- Schließen des betätigbaren Steuerventils für eine vorgegebene Inspirationsdauer und
- Öffnen des betätigbaren Steuerventils für eine vorgegebene Exspirationsdauer, wobei
bei geöffnetem Steuerventil während der Exspirationsdauer der von dem Drucksensor ausgegebene Wert erfasst wird und mit einem vorgegebenen ersten Schwellwert verglichen wird und wobei
eine erste Fehlermeldung erzeugt wird, wenn der ausgegebene Wert den ersten Schwellwert überschreitet.

Mit Hilfe des Drucksensors, der vorzugsweise unmittelbar am Reservoiranschluss vorgesehen ist, kann der Druck nahe dem Reservoir gemessen werden. Wenn das Reservoir bzw. der Handbeatmungsbeutel ein zu kleines Maximalvolumen aufweist, ergibt sich zunächst während der Exspirationsphase am Ende eines Ausatemhubes in diesem Bereich ein Überdruck, der von dem Drucksensor erfasst wird. Wenn dieser Überdruck über einer vorgegebenen Schwelle liegt, wird eine Fehlermeldung für einen Benutzer ausgegeben, wobei aufgrund des Überdrucks klar ist, dass das Maximalvolumen des Reservoirs bzw. Handbeatmungsbeutels nicht ausreichend ist.

Diese Schritte können auch ausgeführt werden, wenn das Beatmungssystem keinen Beatmungsantrieb aufweist oder dieser abgeschaltet ist. Es ist allerdings bevorzugt, wenn die Schritte mit Unterstützung eines Beatmungsantriebs ausgeführt werden. Es ist weiter bevorzugt, wenn der Beatmungsantrieb als ein Radialverdichter ausgeführt ist.

Des Weiteren kann in einer bevorzugten Ausführungsform die Zeitdauer gemessen werden, die nach Beginn der Exspirationsdauer vergeht, bis der von dem Drucksensor ausgegebene Wert den ersten Schwellwert überschreitet.
Je kürzer die Zeitdauer ist, desto größer ist das in dem Reservoir fehlende Volumen, so dass es möglich ist, die Fehlermeldung an den Grad anzupassen, um den das Maximalvolumen des Handbeatmungsbeutels oder Reservoirs zu klein ist.

Außerdem ist es bevorzugt, wenn auch bei geschlossenem Steuerventil während der Inspirationsdauer der von dem Drucksensor ausgegebene Wert erfasst wird und mit einem vorgegebenen zweiten Schwellwert verglichen wird, wobei eine Fehlermeldung erzeugt wird, wenn der ausgegebene Wert den zweiten Schwellwert unterschreitet. Bei dieser Betriebsweise wird ausgenutzt, dass sich bei einem unterdimensionierten Reservoir in der Inspirationsphase am Drucksensor ein negativer Druck einstellt, wenn das gesamte Gas durch den Beatmungsantrieb aus dem Reservoir abgezogen und dem Patientenanschluss durch den inspiratorischen Zweig bereits zugeführt worden ist. Damit ist das Auftreten eines negativen Drucks unterhalb eines vorgegebenen Schwellwerts in der inspiratorischen Phase ebenfalls ein Indikator für einen zu klein gewählten Handbeatmungsbeutel.

In diesem Zusammenhang kann ebenfalls in einer weiter bevorzugten Ausführungsform die Zeitdauer gemessen werden, die nach Beginn der inspiratorischen Phase vergeht, bis der von dem Drucksensor ausgegebene Wert den zweiten Schwellwert unterschreitet. Diese Zeitdauer ist dann ebenfalls ein Maß für den Grad, um den das Reservoir unterdimensioniert ist.

In einer weiteren bevorzugten Ausführungsform weist das Beatmungssystem eine Frischgasversorgung auf, mit der zumindest während der Inspirationsphase absorbierte Gasbestandteile kompensiert werden können.

Schließlich ist es weiterhin bevorzugt, wenn ein Volumenstromsensor und ein Drucksensor im inspiratorischen Zweig vorgesehen sind, so dass die Inspirationsphase und die Exspirationsphase von der Steuereinheit überwacht werden können.

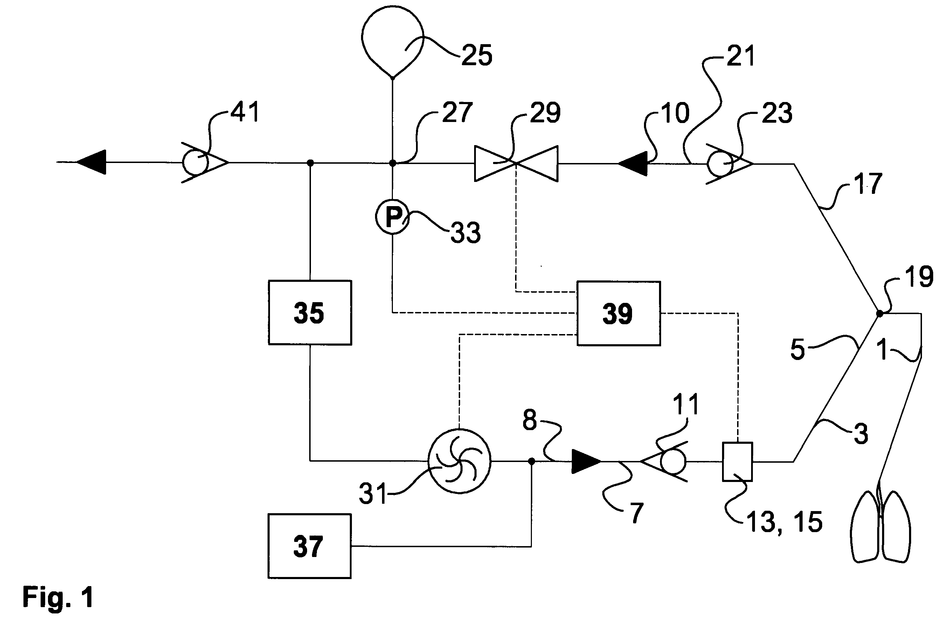

Im Folgenden wird die vorliegende Erfindung anhand einer, -lediglich ein bevorzugtes Ausführungsbeispiel zeigenden-, Zeichnung näher erläutert, wobei die
- Figur 1: einen Gaslaufplan eines Ausführungsbeispiels eines Beatmungssystems zur Ausführung erfindungsgemäßer Schritte zeigt.

Das in Figur 1 dargestellte Beatmungssystem weist zunächst einen Patientenanschluss 1 auf, über den ein Patient mit dem Beatmungssystem verbunden werden kann. Der Patientenanschluss 1 ist als Y-Stück ausgebildet, von dem sich ein inspiratorischer Zweig 3 erstreckt, der ein erstes Ende 5 aufweist, das mit dem Patientenanschluss 1 verbunden ist, und ein zweites Ende 7, mit dem der inspiratorische Zweig 3 mit einem Ausgang 8 einer im Gehäuse des Beatmungssystems vorgesehenen Rückatemleitung 9 verbunden ist, die darüber hinaus noch einen Eingang 10 aufweist.

Schließlich ist in dem inspiratorischen Zweig ein Rückschlagventil 11 vorgesehen, das derart aufgebaut ist, dass es nur dann öffnet, wenn der Druck auf der Seite des Rückschlagventils 11, die zu dem zweiten Ende 7 weist, größer ist als auf der Seite, die zu dem ersten Ende 5 weist. Somit wird sichergestellt, dass Atemgas nicht von dem ersten Ende 5 zu dem zweiten Ende 7 des inspiratorischen Zweigs 3 strömen kann.

Außerdem sind bei dem hier dargestellten bevorzugten Ausführungsbeispiel in dem inspiratorischen Zweig 3 noch ein Volumenstromsensor 13 und ein Drucksensor 15 angeordnet, um die entsprechenden dort auftretenden Parameter der Atemgasströmung erfassen zu können.

Das Beatmungssystem weist ferner einen exspiratorischen Zweig 17 auf, dessen erstes Ende 19 mit dem Patientenanschluss 1 verbunden ist und dessen zweites Ende 21 mit dem Eingang 10 der Rückatemleitung 9 verbunden ist. Außerdem ist in dem exspiratorischen Zweig 17 ebenfalls ein Rückschlagventil 23 vorgesehen, das jedoch derart ausgestaltet ist, dass es nur dann öffnet, wenn der Druck auf der Seite des Rückschlagventils 23, die zu dem ersten Ende 19 weist, größer ist als der Druck auf der Seite, die zu dem zweiten Ende 21 weist. Dadurch ist der exspiratorische Zweig derart ausgestaltet, dass Atemgas darin nur von dem Patientenanschluss 1 weg hin zum Eingang 10 der Rückatemleitung 9 strömen kann, nicht jedoch umgekehrt.

Wie weiter aus Figur 1 hervorgeht, ist in der Rückatemleitung 9 ein Reservoir 25 vorgesehen, das in dieser bevorzugten Ausführungsform als Handbeatmungsbeutel ausgeführt ist und über einen Reservoiranschluss 27 mit der Rückatemleitung 9 verbunden ist. Das Reservoir 25 hat ein variables Volumen mit einem Maximalvolumen, wobei das Volumen bei Druckbeaufschlagung ohne nennenswerten Widerstand bis zum Maximalvolumen vergrößert werden kann. Zwischen dem Reservoiranschluss 27 und dem Eingang 10 der Rückatemleitung 9, ist in der Rückatemleitung 9 ein betätigbares Steuerventil 29 (PEEP-Ventil) vorgesehen, das elektronisch geöffnet oder geschlossen werden kann. Außerdem weist die Rückatemleitung 9 zwischen dem Reservoiranschluss 27 und dem Ausgang 8 einen Beatmungsantrieb 31 auf, der vorzugsweise ein Radialverdichter ist und mit dem eine Strömung in der Rückatemleitung 9 vom Eingang 10 zum Ausgang 8 erzeugt werden kann. Schließlich ist mit dem Reservoiranschluss 27 noch ein Drucksensor 33 verbunden, mit dem der Druck in der Rückatemleitung im Bereich des Reservoiranschlusses 27 gemessen werden kann.

Wie außerdem aus Figur 1 zu erkennen ist, ist in der Rückatemleitung 9 noch ein CO₂-Absorber 35 angebracht, mit dem von dem Patienten abgegebenes CO₂ absorbiert werden kann. Des Weiteren ist eine Frischgasversorgung 37 vorgesehen, mit der der Rückatemleitung 9 Frischgas zugeführt werden kann, wobei dies stromabwärts des Beatmungsantriebs 31 erfolgt.

Um die Komponenten des Beatmungssystems zu steuern, ist schließlich eine Steuereinheit 39 vorgesehen, die mit dem Volumensensorstrom 13, dem Drucksensor 15, dem Steuerventil 29, dem Drucksensor 33 und dem Beatmungsantrieb 31 verbunden ist.

Damit überschüssiges Gas aus der Rückatemleitung 9 austreten kann, ist ein Narkosegasfortleitungsventil ("NGF-Ventil") 41 vorgesehen, das bei einer vorgegebenen Schwelle öffnet.

Das zuvor beschriebene Beatmungssystem arbeitet wie folgt.

Nach einer Exspirationsphase, während derer das Reservoir 25 in Form des Handbeatmungsbeutels gefüllt worden ist, wird das betätigbare Steuerventil 29 durch die Steuereinheit 39 geschlossen, und während der nun folgenden Inspirationsdauer wird in diesem bevorzugten Ausführungsbeispiel der Beatmungsantrieb 31 betrieben, um in der Rückatemleitung 9 eine Strömung vom Reservoir 25 zum zweiten Ende 7 des inspiratorischen Zweigs 3 zu erzeugen, so dass Atemgas aus dem Reservoir 25 durch den CO₂-Absorber 35 hindurchgezogen und dem Patientenanschluss 1 zugeführt wird.

Während der Inspirationsdauer wird der von dem Drucksensor 33 ausgegebene Wert von der Steuereinheit 39 erfasst und mit einem vorgegebenen zweiten Schwellwert verglichen. Unterschreitet der ausgegebene Wert den zweiten Schwellwert, wird eine Fehlermeldung ausgegeben. Außerdem wird die Zeitdauer gemessen, die zwischen dem Schließen des betätigbaren Steuerventils 29 und dem Zeitpunkt, bei dem der vom Drucksensor 33 ausgegebene Wert den zweiten Schwellwert unterschreitet, liegt.

Der zweite Schwellwert ist so gewählt, dass ein Unterschreiten dieses Werts bedeutet, dass sich im Bereich des Reservoiranschlusses 27 ein Unterdruck entwickelt hat, der darauf zurückzuführen ist, dass das Reservoir 25 vollständig entleert worden ist, obwohl die Inspirationsdauer noch nicht erreicht ist. Abhängig von der gemessenen Zeitdauer zwischen dem Beginn der Inspirationsdauer und dem Unterschreiten des zweiten Schwellwerts variiert die Fehlermeldung, wobei eine kurze Zeit darauf hinweist, dass das Volumen des Reservoirs 25 erheblich zu klein ist, während eine vergleichsweise lange Zeitdauer, die nur etwas geringer als die Dauer der Inspirationsphase ist, darauf hinweist, dass das Volumen des Reservoirs 25 nur geringfügig zu klein ist. Dementsprechend wird die Fehlermeldung angepasst.

Anschließend wird das betätigbare Steuerventil 29 geöffnet, und im hier beschriebenen bevorzugten Ausführungsbeispiel wird der Beatmungsantrieb 31 mit verringerter Leistung betrieben, so dass das Rückschlagventil 11 im Inspirationszweig 3 geschlossen bleibt. Während der Exspirationsdauer strömt Atemgas durch den exspiratorischen Zweig 17, das Steuerventil 29 und den Reservoiranschluss 27 in das Reservoir 25, das sich dabei kontinuierlich füllt. Dabei wird wiederum der Druck am Reservoiranschluss 27 mit Hilfe des Drucksensors 33 erfasst, wobei der von dem Drucksensor 33 ausgegebene Wert in der Steuereinheit 39 mit einem ersten Schwellwert verglichen wird und eine Fehlermeldung dann ausgegeben wird, wenn der ausgegebene Wert den ersten Schwellwert überschreitet.

Der erste Schwellwert ist dabei so gewählt, dass ein Überschreiten dieses Werts bedeutet, dass sich im Bereich des Reservoiranschlusses 27 ein Überdruck ausbildet, der dadurch zustande kommt, dass das Reservoir 25 vollständig gefüllt ist und nur noch durch Überwindung eines Widerstands, der beispielsweise durch die Elastizität des Handbeatmungsbeutels zustande kommt, weiter gefüllt werden kann. Wenn dies eintritt, wird das weitere vom Patientenanschluss kommende Atemgas über das NGF-Ventil 41 abgelassen.

Wenn sich jedoch im Bereich des Reservoiranschlusses 27 kein Druck aufbaut, der den ersten Schwellwert überschreitet, weist dies darauf hin, dass das Volumen des Reservoirs 25 ausreichend groß ist, und es wird keine Fehlermeldung ausgegeben.

Auch während der Exspirationsdauer wird die Zeitdauer erfasst, die zwischen dem Beginn der Exspirationsdauer, also dem Schließen des Steuerventils 29, und dem Überschreiten des ersten Schwellwerts durch den von dem Drucksensor 33 ausgegebenen Wert liegt. Je kürzer diese Zeitdauer ist, in umso größerem Maße ist das Maximalvolumen des Reservoirs 25 unterdimensioniert, und die ausgegebenen Fehlermeldung wird abhängig von dieser Zeitdauer angepasst.

Das zuvor beschriebene Ausführungsbeispiel ist mit einem Beatmungsantrieb 31 versehen. Die erfindungsgemäßen Schritte können jedoch auch ohne einen solchen Beatmungsantrieb durch Schalten des steuerbaren Ventils 29 ausgeführt werden. In diesem Fall ist es dann vorteilhaft, wenn im Inspirationszweig 3 der Volumenstromsensor 13 und der Drucksensor 15 vorhanden sind, so dass die entsprechenden Parameter durch die Steuereinheit 39 erfasst werden können.

Um zu prüfen, ob das Volumen des Reservoirs 25 ausreichend ist, ist es lediglich erforderlich, während der Exspirationsphase den Druck im Bereich des Reservoiranschlusses 27 zu erfassen, um einen sich dort aufbauenden Überdruck messen zu können, der auf eine zu geringe Kapazität des Reservoirs 25 hinweist, so dass dann eine Fehlermeldung ausgegeben werden kann, aufgrund derer der Bediener dann ggf. ein anderes Reservoir einsetzt, das die erforderliche Kapazität hat.

Mit dem erfindungsgemäßen Beatmungssystem ist es also möglich, in einfacher Weise festzustellen, dass das Beatmungssystem fehlerhaft dimensioniert ist. Dabei ermöglicht die Messung der Zeitdauer zwischen dem Beginn der Inspirations- bzw. Exspirationsphase und dem Überschreiten bzw. Unterschreiten des jeweiligen Schwellwerts, den Umfang festzustellen, in dem das Volumen des Reservoirs 25 zu klein ist.

### Bezugsziffernliste

- 1: Patientenanschluss, Y- Stück
- 3: Inspiratorischer Zweig
- 5: erstes Ende (Inspiratorischer Zweig)
- 7: zweites Ende (Inspiratorischer Zweig)
- 8: Ausgang der Rückatemleitung
- 9: Rückatemleitung
- 10: Eingang der Rückatemleitung
- 11: Rückschlagventil (Inspiratorischer Zweig)
- 13: Volumenstromsensor (Inspiratorischer Zweig)
- 15: Drucksensor (Inspiratorischer Zweig)
- 17: Exspiratorischer Zweig
- 19: erstes Ende (Exspiratorischer Zweig)
- 21: zweites Ende (Exspiratorischer Zweig)
- 23: Rückschlagventil (Exspiratorischer Zweig)
- 25: Reservoir (Handbeatmungsbeutel)
- 27: Reservoiranschluss
- 29: Steuerventil
- 31: Beatmungsantrieb (Radialverdichter)
- 33: Drucksensor (Reservoir)
- 35: CO₂- Absorber
- 37: Frischgasversorgung
- 39: Steuereinheit 39
- 41: Narkosegasfortleitungsventil (NGF-Ventil)

## Patentansprüche

1. Beatmungssystem mit
einem Patientenanschluss (1),
einem inspiratorischen Zweig (3), der ein erstes und ein zweites Ende (5, 7) sowie ein Rückschlagventil (11) aufweist, das dann öffnet, wenn der Druck auf der zum zweiten Ende (7) weisenden Seite des Rückschlagventils (11) über dem auf der zum ersten Ende (5) weisenden Seite des Rückschlagventils (11) liegt, wobei das erste Ende (5) mit dem Patientenanschluss (1) verbunden ist,
einem exspiratorischen Zweig (17), der ein erstes und ein zweites Ende (19, 21) sowie ein Rückschlagventil (23) aufweist, das dann öffnet, wenn der Druck auf der zum ersten Ende (19) weisenden Seite des Rückschlagventils (23) über dem auf der zum zweiten Ende (21) weisenden Seite des Rückschlagventils (23) liegt, wobei das erste Ende (19) mit dem Patientenanschluss (1) verbunden ist,
einer Rückatemleitung (9), die das zweite Ende (7) des inspiratorischen Zweigs (3) mit dem zweiten Ende (21) des exspiratorischen Zweigs (17) verbindet,
einem Reservoir (25), das ein variables Volumen mit einem Maximalvolumen hat und das mit einem Reservoiranschluss (27) in der Rückatemleitung (9) verbunden ist,
einem betätigbaren Steuerventil (29), das in der Rückatemleitung (9) zwischen dem zweiten Ende (21) des exspiratorischen Zweigs (17) und dem Reservoiranschluss (27) vorgesehen ist,
einem Drucksensor (33), der benachbart zu dem Reservoiranschluss (27) mit der Rückatemleitung (9) verbunden ist, und
einer Steuereinheit (39), die mit dem betätigbaren Steuerventil (29) und mit dem Drucksensor (33) verbunden ist,
**dadurch gekennzeichnet, dass**
das Beatmungssystem angepasst ist, um die folgenden Schritte auszuführen:
Schließen des betätigbaren Steuerventils (29) für eine vorgegebene Inspirationsdauer und Öffnen des betätigbaren Steuerventils (29) für eine vorgegebene Exspirationsdauer,
wobei bei geöffnetem Steuerventil (29) während der Exspirationsdauer der von dem Drucksensor (33) ausgegebene Wert erfasst wird und mit einem vorgegebenen ersten Schwellwert verglichen wird und
wobei eine erste Fehlermeldung erzeugt wird, wenn der ausgegebene Wert den ersten Schwellwert überschreitet.

2. Beatmungssystem nach Anspruch 1, wobei das Beatmungssystem einen Beatmungsantrieb (31) aufweist, der in der Rückatemleitung (9) zwischen dem Reservoiranschluss (27) und dem zweiten Ende (21) des inspiratorischen Zweigs (17) angeordnet ist,
wobei das Beatmungssystem angepasst ist, um während der Inspirationsdauer den Beatmungsantrieb (31) zu betreiben, sodass in der Rückatemleitung (9) eine Strömung vom Reservoir (25) zum zweiten Ende (7) des inspiratorischen Zweigs (3) erzeugt wird, und während der Exspirationsdauer den Beatmungsantrieb (31) abzuschalten oder mit verringerter Leistung zu betreiben, sodass das Rückschlagventil (11) im inspiratorischen Zweig (3) geschlossen bleibt.

3. Beatmungssystem nach Anspruch 1 oder 2, wobei das Beatmungssystem angepasst ist,
um die Zeitdauer zwischen dem Öffnen des Steuerventils (29) und dem Zeitpunkt zu messen, bei dem der von dem Drucksensor (33) ausgegebene Wert den ersten Schwellwert überschreitet, und
um die erste Fehlermeldung in Abhängigkeit von der gemessenen Zeitdauer zu variieren.

4. Beatmungssystem nach einem der Ansprüche 1 bis 3, wobei das Beatmungssystem angepasst ist,
um bei geschlossenem Steuerventil (29) während der Inspirationsdauer den von dem Drucksensor (33) ausgegebenen Wert zu erfassen und mit einem vorgegebenen zweiten Schwellwert zu vergleichen, und
um eine Fehlermeldung zu erzeugen, wenn der ausgegebene Wert den zweiten Schwellwert unterschreitet.

5. Beatmungssystem nach Anspruch 4, wobei das Beatmungssystem angepasst ist,
um die Zeitdauer zwischen dem Schließen des Steuerventils (29) und dem Zeitpunkt zu messen, bei dem der von dem Drucksensor (33) ausgegebene Wert den zweiten Schwellwert unterschreitet, und
um die zweite Fehlermeldung in Abhängigkeit von der gemessenen Zeitdauer zu variieren.

6. Beatmungssystem nach einem der Ansprüche 1 bis 5, wobei das Reservoir (25) als Handbeatmungsbeutel ausgebildet ist.

7. Beatmungssystem nach einem der Ansprüche 1 bis 6, wobei das Beatmungssystem eine mit der Rückatemleitung (9) verbundene Frischgasversorgung (37) aufweist, mit der Frischgas in die Rückatemleitung (9) eingespeist werden kann.

8. Beatmungssystem nach einem der Ansprüche 1 bis 7, wobei im inspiratorischen Zweig (3) ein Volumenstromsensor (13) und ein Drucksensor (15) vorgesehen sind und
wobei das Beatmungssystem angepasst ist, um den Druck und den Volumenstrom während der Inspirationsdauer und der Exspirationsdauer zu erfassen.

9. Beatmungssystem nach einem der Ansprüche 1 bis 8, wobei der Drucksensor (33) am Reservoiranschluss (27) angeordnet ist.

## Claims

1. A respiration system having
a patient connection (1),
an inspiratory branch (3) which has a first and a second end (5, 7) and also a non-return valve (11) that opens when the pressure on the side of the non-return valve (11) pointing to the second end (7) lies above that on the side of the non-return valve (11) pointing to the first end (5), wherein the first end (5) is connected to the patient connection (1),
an expiratory branch (17) which has a first and a second end (19, 21) and also a non-return valve (23) that opens when the pressure on the side of the non-return valve (23) pointing to the first end (19) lies above that on the side of the non-return valve (23) pointing to the second end (21), wherein the first end (19) is connected to the patient connection (1),
a rebreathing line (9) which connects the second end (7) of the inspiratory branch (3) to the second end (21) of the expiratory branch (17),
a reservoir (25) which has a variable volume with a maximum volume and which is connected to a reservoir connection (27) in the rebreathing line (9),
an actuatable control valve (29) which is provided in the rebreathing line (9) between the second end (21) of the expiratory branch (17) and the reservoir connection (27),
a pressure sensor (33) which is connected to the rebreathing line (9) adjacently to the reservoir connection (27), and
a control unit (39) which is connected to the actuatable control valve (29) and to the pressure sensor (33),
**characterised in that**
the respiration system is adapted to carry out the following steps:
closure of the actuatable control valve (29) for a predetermined period of inspiration and opening of the actuatable control valve (29) for a predetermined period of expiration,
wherein when the control valve (29) is opened during the period of expiration the value output by the pressure sensor (33) is detected and compared with a predetermined first threshold value, and
wherein a first error message is generated when the value output exceeds the first threshold value.

2. A respiration system according to claim 1, wherein the respiration system has a respiration drive (31) which is arranged in the rebreathing line (9) between the reservoir connection (27) and the second end (21) of the inspiratory branch (17),
wherein the respiration system is adapted to operate the respiration drive (31) during the period of inspiration so that a flow is generated in the rebreathing line (9) from the reservoir (25) to the second end (7) of the inspiratory branch (3) and during the period of expiration to switch off the respiration drive (31) or to operate it at reduced capacity so that the non-return valve (11) in the inspiratory branch (3) remains closed.

3. A respiration system according to claim 1 or 2, wherein the respiration system is adapted
to measure the period of time between the opening of the control valve (29) and the point in time at which the value output by the pressure sensor (33) exceeds the first threshold value, and
to vary the first error message as a function of the period of time measured.

4. A respiration system according to one of claims 1 to 3,
wherein the respiration system is adapted
to detect the value output by the pressure sensor (33) when the control valve (29) is closed during the period of inspiration and to compare it with a predetermined second threshold value, and
to generate an error message when the value output falls below the second threshold value.

5. A respiration system according to claim 4, wherein the respiration system is adapted to
measure the period of time between the closure of the control valve (29) and the point in time at which the value output by the pressure sensor (33) falls below the second threshold value, and
to vary the second error message as a function of the period of time measured.

6. A respiration system according to one of claims 1 to 5,
wherein the reservoir (25) is formed as a manual respiration bag.

7. A respiration system according to one of claims 1 to 6,
wherein the respiration system has a fresh-gas supply (37) which is connected to the rebreathing line (9) and with which fresh gas can be fed into the rebreathing line (9).

8. A respiration system according to one of claims 1 to 7,
wherein a volume-flow sensor (13) and a pressure sensor (15) are provided in the inspiratory branch (3), and
wherein the respiration system is adapted to detect the pressure and the volume flow during the period of inspiration and the period of expiration.

9. A respiration system according to one of claims 1 to 8,
wherein the pressure sensor (33) is arranged at the reservoir connection (27).

## Revendications

1. Système de ventilation comprenant
un raccordement (1) à un patient,
un embranchement inspiratoire (3) comportant des première et seconde extrémités (5, 7), ainsi qu'un clapet antiretour (11) qui s'ouvre lorsque la pression, régnant du côté du clapet antiretour (11) tourné vers la seconde extrémité (7), est supérieure à celle régnant du côté dudit clapet antiretour (11) tourné vers la première extrémité (5), ladite première extrémité (5) étant reliée au raccordement (1) au patient,
un embranchement expiratoire (17) comportant des première et seconde extrémités (19, 21), ainsi qu'un clapet antiretour (23) qui s'ouvre lorsque la pression, régnant du côté du clapet antiretour (23) tourné vers la première extrémité (19), est supérieure à celle régnant du côté dudit clapet antiretour (23) tourné vers la seconde extrémité (21), ladite première extrémité (19) étant reliée audit raccordement (1) au patient,
un conduit de réinspiration (9) reliant la seconde extrémité (7) de l'embranchement inspiratoire (3) à la seconde extrémité (21) de l'embranchement expiratoire (17),
un réservoir (25) qui présente un volume variable à volume maximal, et est relié à un raccord (27) dans ledit conduit de réinspiration (9),
une vanne actionnable de commande (29) prévue, dans ledit conduit de réinspiration (9), entre la seconde extrémité (21) de l'embranchement expiratoire (17) et le raccord (27) du réservoir,
un capteur de pression (33) connecté audit conduit de réinspiration (9) au voisinage dudit raccord (27) du réservoir, et
une unité de commande (39) connectée à ladite vanne actionnable de commande (29) et audit capteur de pression (33),
**caractérisé par le fait que**
ledit système de ventilation est adapté pour exécuter les étapes suivantes :
fermeture de la vanne actionnable de commande (29) pour une durée d'inspiration préétablie, et ouverture de ladite vanne actionnable de commande (29) pour une durée d'expiration préétablie,
sachant que, lorsque ladite vanne de commande (29) est à l'état ouvert, pendant ladite durée d'expiration, la valeur délivrée par le capteur de pression (33) est saisie et comparée à une première valeur de seuil préétablie, et
sachant qu'une première notification d'erreur est engendrée lorsque ladite valeur délivrée excède ladite première valeur de seuil.

2. Système de ventilation selon la revendication 1, ledit système de ventilation étant équipé d'un entraînement ventilatoire (31) logé dans le conduit de réinspiration (9), entre le raccord (27) du réservoir et la seconde extrémité (21) de l'embranchement expiratoire (17),
sachant que ledit système de ventilation est adapté pour actionner ledit entraînement ventilatoire (31) pendant la durée d'inspiration, de telle sorte qu'une circulation soit instaurée, dans ledit conduit de réinspiration (9), depuis le réservoir (25) jusqu'à la seconde extrémité (7) de l'embranchement inspiratoire (3), et pour mettre ledit entraînement ventilatoire (31) à l'arrêt, ou pour l'actionner avec puissance réduite pendant la duré d'expiration, de façon telle que le clapet antiretour (11) demeure fermé dans ledit embranchement inspiratoire (3).

3. Système de ventilation selon la revendication 1 ou 2, ledit système de ventilation étant adapté
pour mesurer le laps de temps compris entre l'ouverture de la vanne de commande (29) et l'instant auquel la valeur, délivrée par le capteur de pression (33), excède la première valeur de seuil, et
pour faire varier la première notification d'erreur en fonction dudit laps de temps mesuré.

4. Système de ventilation selon l'une des revendications 1 à 3, ledit système de ventilation étant adapté
pour saisir la valeur délivrée par le capteur de pression (33) pendant la durée d'inspiration, à l'état fermé de la vanne de commande (29), et pour la comparer à une seconde valeur de seuil préétablie, et
pour engendrer une notification d'erreur lorsque ladite valeur délivrée dépasse négativement ladite seconde valeur de seuil.

5. Système de ventilation selon la revendication 4, ledit système de ventilation étant adapté
pour mesurer le laps de temps compris entre la fermeture de la vanne de commande (29) et l'instant auquel la valeur, délivrée par le capteur de pression (33), dépasse négativement la seconde valeur de seuil, et
pour faire varier la seconde notification d'erreur en fonction dudit laps de temps mesuré.

6. Système de ventilation selon l'une des revendications 1 à 5, dans lequel le réservoir (25) est réalisé sous la forme d'un sac de ventilation manuelle.

7. Système de ventilation selon l'une des revendications 1 à 6, ledit système de ventilation étant équipé d'une alimentation (37) en gaz frais qui est raccordée au conduit de réinspiration (9), et par laquelle du gaz frais peut être introduit dans ledit conduit de réinspiration (9).

8. Système de ventilation selon l'une des revendications 1 à 7, dans lequel un capteur de débit (13) et un capteur de pression (15) sont prévus dans l'embranchement inspiratoire (3),
sachant que ledit système de ventilation est adapté à la détection de la pression et du débit pendant la durée d'inspiration et la durée d'expiration.

9. Système de ventilation selon l'une des revendications 1 à 8, dans lequel le capteur de pression (33) est implanté sur le raccord (27) du réservoir.
